## Europäisches Patentamt

## European Patent Office

## Office européen des brevets

(19)

(11) Veröffentlichungsnummer: **0 164 579**
**B1**

(12) # EUROPÄISCHE PATENTSCHRIFT

(45) Veröffentlichungstag der Patentschrift:
02.08.89

(51) Int. Cl.⁴: **A 61 K 7/16,** C 01 F 11/18

(21) Anmeldenummer: 85105711.7

(22) Anmeldetag: 09.05.85

(54) Mittel zur Anhebung des pH-Werts der Zahnplaque und Verfahren zu seiner Herstellung.

(30) Priorität: 10.05.84 DE 3417393

(43) Veröffentlichungstag der Anmeldung:
18.12.85 Patentblatt 85/51

(45) Bekanntmachung des Hinweises auf die Patenterteilung:
02.08.89 Patentblatt 89/31

(84) Benannte Vertragsstaaten:
AT BE CH DE FR GB IT LI LU NL SE

(73) Patentinhaber: Neutralith Antikaries GmbH &
CO.KG, Hauenhorster Strasse 147, D-4440 Rheine
1 (DE)

(72) Erfinder: Knappwost, Adolf, Prof. Dr., Am
Pfarrgarten 3, D-3220 Alfeld (DE)

(74) Vertreter: Patentanwälte TER MEER - MÜLLER -
STEINMEISTER, Mauerkircherstrasse 45, D-8000
München 80 (DE)

(56) Entgegenhaltungen:
FR-A-2 377 969
FR-A-2 433 481

"Cosmetics Science and Technology", 2. Auflage,
Band 1, 1972, Seiten 461-477, Wiley-Interscience,
New York, US;
CHEMICAL ABSTRACTS, Band 63, 22. November
1965, Spalte 14409-d, Columbus, Ohio, US; R.
MALLIKARJUNAN u.a.: "The effect of some
surface-active reagents on the comminution of
quartz and calcite" & TRANS. INDIAN INST.
METALS 18, 79-82 (1965)
CHEMICAL ABSTRACTS, Band 79, 17. Dezember
1973, Seite 223, Nr. 140443c, Columbus, Ohio, US; E.
FRIES u.a.: "X-ray diffraction study of the effects
of milling on calcite" & BULL. SOC. FR. MINERAL.
CRISTAL-LOGR. 1973, 96(2), 91-6
CHEMICAL ABSTRACTS, Band 74, 11. Januar 1971,

(56) Entgegenhaltungen: (Fortsetzung)
Seite 274, Nr. 7351b, Columbus, Ohio, US; J.L.
DANDURAND: "Establishment of a calcitearagonite equilibrium by grinding" & C.R. ACAD.
SCI., SER. D 1970, 271(11), 881-3
C.R. Acad. Sc., Paris, 271 (1970), pp. 881-883
Bull. Soc. fr. Mineral Cristallog, (1973), 96, pp. 91-96

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Mittel zur Anhebung des pH-Werts der Zahnplaque und ein Verfahren zu seiner Herstellung.

Es ist bekannt, daß die Karies durch die Anlösung der mineralischen Zahnhartsubstanz Hydroxylapatit [$Ca_{10}(PO_4)_6$-$(OH)_2$] verursacht wird, die durch organische Säuren (Milchsäure, Essigsäure, Ameisensäure, etc.) bewirkt wird, die als Stoffwechselprodukte bei der enzymatischen Umsetzung von gelösten Kohlenhydraten wie Saccharose, Glucose etc., durch azidogene Bakterien in der Zahnplaque entstehen. Nach dem Genuß von beispielsweise Saccharose oder Glucose oder von im Speichel aufschließbarer Stärke setzt die Bildung dieser Säuren innerhalb weniger Minuten ein, so daß im Verlaufe von etwa 20 bis 30 Minuten der pH-Wert innerhalb der Zahnplaque auf Werte von 5 und häufig noch niedriger absinkt. Mit sinkendem pH-Wert steigt aber die Löslichkeit des Hydroxylapatits in Wasser stark an. Sie liegt bei 37°C nach eigenen Messungen bei einem pH-Wert von 7,0 bei 12,5 mg/l und erreicht bei einem pH-Wert von 5,0 den Wert von 628 mg/l. Die hohe Löslichkeit ist bei diesen niedrigen pH-Werten durch eine Remineralisation durch die im Speichel vorhandenen Calcium-, Phosphat- und Hydroxylionen nicht zu kompensieren, da die Hydroxylionenkonzentration ebenso wie die Phosphatkonzentration um mehrere Zehnerpotenzen unter den für die Überschreitung des Löslichkeitsprodukts des Hydroxylapatits erforderlichen Werten liegen.

Im Wechselspiel zwischen der Lösungsgeschwindigkeit $V_L$ und der Remineralisierungsgeschwindigkeit $V_{Rem}$ des Hydroxylapatits überwiegt dann die erstere, so daß die Bedingung $V_L > V_{Rem}$, die nach der Remineralisationstheorie der Karies eine progressive Karies bedeutet, erfüllt ist.

Es ist andererseits bekannt, daß eine Remineralisation der Zahnhartsubstanz unterhalb der Zahnplaque durch eine relativ hohe Konzentration von Fluoridionen ($\geq 10^{-3}$ Mol/l) im Speichel erzwungen werden kann, da die Fluoridionen die Hydroxylionen im Apatit ersetzen können. Jedoch stehen der peroralen Aufnahme von Fluoriddosen, die eine solche Fluoridkonzentration im Speichel bewirken, vielfach Schwierigkeiten entgegen.

Die Aufgabe der vorliegenden Erfindung besteht nun darin, ein Mittel anzugeben, mit dem es gelingt, den pH-Wert in der Zahnplaque nach Aufnahme von vergärbaren Kohlenhydraten nur schwach oder gar nicht absinken oder sogar schwach ansteigen zu lassen, um in dieser Weise die oben angesprochene Lösungsgeschwindigkeit $V_L$ drastisch herabzusetzen und die Remineralisation des Zahnschmelzes zu bewirken.

Man könnte natürlich daran denken, die fraglichen Nahrungs- oder Genußmittel mit wenigen Prozent eines säurebindenden Stoffs zu versetzen. Hierfür scheiden aber gelöst alkalische Stoffe, beispielsweise Salze, wegen ihres unangenehmen Geschmacks aus. Daher kommen nur schwerlösliche Stoffe in Betracht, die sich schnell in den Säuren lösen. Versuche mit handelsüblichem gefälltem Calciumcarbonat ergaben jedoch bei der Verfolgung der Änderung des pH-Werts in der Zahnplaque mit Hilfe einer pH-empfindlichen Mikroelektrode keinen hinreichenden Effekt; abgesehen davon, daß sich auch geschmackliche Nachteile feststellen ließen.

Es ist weiterhin bereits bekannt, daß die spezifische Oberfläche von gefälltem neutralisierendem Pulver größer als 6 m²/g sein muß, wenn ein solches Pulver durch die Strömung beim Kauakt in hinreichenden Mengen in die Zahnplaque eindringen soll. Durch die Carbonatisierung von aufgeschlämmtem Calciumhydroxid ist eine solch große spezifische Oberfläche aus physikalisch-chemischen Gründen (Kelvin-Gleichung) nicht zu erreichen. Es gelang jedoch, durch plötzliches Zusammenbringen von stärker konzentrierten Lösungen von Calciumnitrat und Ammoniumcarbonat in Gegenwart eines Tensids und von Schutzkolloiden als Kristallwachstumshemmer Calciumcarbonat mit einer spezifischen Oberfläche von mehr als 10 m²/g durch Fällung zu gewinnen. Wenn man dieses hochdisperse Calciumcarbonat in einer Menge von etwa 1 Gew.-% den kariogenen Stoffen (Puderzucker, Gebäck, Brot) zusetzt, so ergibt sich im Vergleich zu dem undotierten Material kaum eine Absenkung des pH-Werts der Zahnplaque. Darüber hinaus ist die Herstellung größerer Mengen des nach diesem oder ähnlichen Fällungsverfahren gebildeten hochdispersen Calciumcarbonats wegen der erforderlichen Filtration und der Notwendigkeit, das gefällte Material durch mehrmaliges Waschen von den bei der Fällung entstandenen Salzen sowie vom Tensid weitgehend zu befreien, schwierig und unwirtschaftlich.

Es hat sich nun überraschenderweise gezeigt, daß hochdisperse, schwerlösliche Salze aus einem oder mehreren Erdalkalimetallelementen und einer schwachen Säure mit einer bestimmten spezifischen Oberfläche und einer bestimmten Gitterfehlerkonzentration eine überraschend starke Wirkung auf die Absenkung des pH-Werts der Zahnplaque ausüben.

Die oben angesprochene Aufgabe wird daher gelöst durch das erfindungsgemäße Mitte.

Gegenstand der Erfindung ist daher ein Mittel zur Anhebung des pH-Werts der Zahnplaque bzw. der Zahnbeläge, welches gekennzeichnet ist durch en Gehalt mindestens eines hochdispersen, schwerlöslichen Erdalkalioxids, Erdalkalihydroxids oder physiologisch unbedenklichen Salzes aus einem oder mehreren Erdkalialielèmenten und solchen Säuren, die mit Milchsäure ein Puffersystem bilden, dessen pH-Wert größer als 5,7 ist, jeweils mit einer spezifischen Oberfläche von mehr als 6 m²/g und einer Gitterfehlerkonzentration, die einer Halbwertsbreite der Röntgeninterferenzen von Cu-Kα-

Strahlung in Glanzwinkelbereich von 20 bis 23° von mehr als 0,25 Winkelgraden entspricht.

Vorzugsweise besitzen die in dem erfindungsgemäßen Mittel enthaltenen Erdalkaliverbindungen eine spezifische Oberfläche von mehr als 10 m²/g und eine Gitterfehlerkonzentration, die einer Halbwertbreite der Röntgeninterferenzen von Cu-Kα-Strahlung im Glanzwinkelbereich von 20° bis 23° von mehr als 0,30 Winkelgraden entspricht.

Dabei entspricht eine spezifische Oberfläche des $CaCO_3$ von 10 m²/g einer mittleren Teilchengröße (kubische Äquivalentseitenkante) von a = 0,22 μm. Die Beziehung zwischen dieser Teilchengröße a und der spezifischen Oberfläche $O_{spez.}$ ist gegeben durch $a = \frac{6}{O_{spez.} \cdot \rho}$, wobei ρ für die Dichte des Materials steht.

Erfindungsgemäß werden als Erdalkaliverbindungen sämtliche physiologisch unbedenklichen Erdalkalisalze solcher Säuren verwendet, die mit Milchsäure ein Puffersystem bilden, dessen pH-Wert größer als 5,7 ist. Deshalb scheiden die bereits vorgeschlagenen Erdalkaliphosphate aus, die mit Milchsäure ein Puffersystem bilden, dessen pH-Wert bei etwa 4,5 liegt. Vorzugsweise enthält das Mittel der Erfindung daher als Erdalkaliverbindung Carbonate, Silicate, Lactate, Tartrate, Aluminate und/oder insbesondere durch Hochtemperaturbehandlung gealterte Hydroxide und Oxide von Calcium und/oder Magnesium oder Doppelsalze auf der Grundlage dieser Verbindungen.

Das erfindungsgemäße Mittel kann die hochdispersen Erdalkaliverbindungen als einzigen Bestandteil enthalten oder kann daneben noch übliche Bindemittel, Trägermaterialien, Hilfsstoffe und/oder Streckmittel enthalten, die keine kariogene Wirkung entfalten und lebensmittelrechtlich zulässig sind. Das erfindungsgemäße Mittel wird bei seiner bestimmungsgemäßen Verwendung vorzugsweise den Nahrungs- oder Genußmitteln zugesetzt, beispielsweise dem bei der Herstellung solcher Produkte verwendeten Zucker oder sonstigen Kohlenhydraten, Backwaren und insbesondere Süß- und Zuckerwaren, wie Hartkaramellen, Weichkaramellen, Schokolade und dergleichen. Dabei muß darauf geachtet werden, daß im Fall von Nahrungs- oder Genußmitteln, die überwiegend durch Lutschen genossen werden, die Teilchengröße der Erdalkalimetallsalze ausreichend gering ist, um eine Beeinträchtigung der organoleptischen Eigenschaften des mit dem erfindungsgemäßen Mittel behandelten Produkts zu vermeiden.

Die in dem erfindungsgemäßen Mittel enthaltenen hochdispersen Erdalkaliverbindungen, beispielsweise Calciumcarbonat, Magnesiumcarbonat oder Doppelsalze dieser Erdalkalicarbonate können durch Naßmahlung der natürlichen Materialien in einer nichtwäßrigen Flüssigkeit, um ein Teilchengrößenwachstum durch sogenannte Ostwald-Reifung beim Abtrenn- und Trocknungsprozeß zu vermeiden, hergestellt werden. Die hierbei erhaltenen Produkte besitzen spezifische Oberflächen von etwa 15 m²/g und entfalten bezüglich der Anhebung des pH-Werts der Zahnplaque eine gute Wirkung.

Die hierin angesprochene spezifische Oberfläche der hochdispersen Erdalkalimetallsalze wird nach der BET-Methode bestimmt.

Da, wie sich gezeigt hat, die Geschwindigkeit der Reaktion der hochdispersen Pulver mit den in der Zahnplaque vorhandenen Säuren nicht nur von der spezifischen Oberfläche der Pulver abhängt, sondern mit der Konzentration der verschiedenen Gitterfehler zunimmt, hat es sich jedoch als vorteilhaft erwiesen, eine höhere Gitterfehlerkonzentration zu erreichen. Es konnte festgestellt werden, daß dies mit Hilfe einer stärkeren Stoßbeanspruchung des Mahlguts durch trockenes Vermahlen erreicht werden kann. Die erzielte Gitterfehlerkonzentration spiegelt sich nun in einer Verbreiterung der Röntgeninterferenzen wider. Eine solche ist auch mit abnehmender Teilchengröße zu beobachten. Allerdings können die durch die Teilchengröße einerseits und die Gitterfehlerkonzentration andererseits auf die Verbreiterung der Röntgeninterferenzen bewirkten Effekte leicht voneinander unterschieden werden, da insbesondere der Effekt der Gitterfehler in diesem Teilchengrößenbereich (0,1 μm) wesentlich größer ist als der auf der Teilchengröße bzw. der spezifischen Oberfläche beruhende. Der Gitterfehlereffekt wird dadurch quantitativ erfaßt, daß er bei Temperung der Verbindungen im abgeschlossenen Rohr bei 750° C für 2 Stunden völlig aufgehoben wird.

Gegenstand der Erfindung ist daher auch ein Verfahren zur Herstellung der in dem erfindungsgemäßen Mittel enthaltenen hochdispersen Erdalkaliverbindungen, welches darin besteht, die Erdalkaliverbindungen bis zum Erreichen einer Gitterfehlerkonzentration, die einer Halbwertsbreite der Röntgeninterferenzen von Cu-Kα-Strahlung im Glanzwinkelbereich von 20° bis 23° von mehr als 0,25 Winkelgraden entspricht, trocken zu vermahlen. Bei diesem Vermahlen stellt sich auch die erfindungswesentliche spezifische Oberfläche von mehr als 6 m²/g ein. So erreicht man beim trockenen Vermahlen von Calciumcarbonat (Calcit) in einer Kugel- oder Schwingmühle bei einer Mahldauer von 120 Minuten ohne weiteres eine spezifische Oberfläche von 9 m²/g und eine Halbwertsbreite der Röntgeninterferenzen unter den genannten Bedingungen von etwa 0,40 Winkelgraden. Die in dieser Weise erhaltenen Produkte entsprechen hinsichtlich ihrer pH-steigernden Wirkung auf die Zahnplaque den naßvermahlenen Produkten mit einer spezifischen Oberfläche von 16 m²/g, die eine wesentlich geringere Gitterfehlerkonzentration aufweisen.

Es hat sich weiterhin überraschenderweise gezeigt, daß eine um etwa 30 % größere spezifische Oberfläche bei sonst gleichen Mahlbedingungen erreicht werden kann, wenn das trockene Vermahlen in Luft bzw. Stickstoff erfolgt, wobei diesen Gasen die Dämpfe von reinen polaren Flüssigkeiten mit einem Siedepunkt von

unterhalb 130°C, wie Wasser oder organische Flüssigkeiten, zugesetzt werden.

Gegenstand der Erfindung ist daher auch eine besonders bevorzugte Ausführungsform des oben angesprochenen Verfahrens, welches dadurch gekennzeichnet ist, daß das trockene Vermahlen in einer Atmosphäre bewirkt wird, die neben Luft oder Stickstoff die Dämpfe reiner polarer Flüssigkeiten mit einem Siedepunkt unterhalb 130°C, und vorzugsweise unterhalb 105°C, oder von Mischungen solcher polarer Flüssigkeiten enthalten. Vorzugsweise verwendet man als solche polare Flüssigkeiten Wasser oder organische Flüssigkeiten, wie niedermolekulare Alkohole mit vorzugsweise 1 bis 5 Kohlenstoffatomen, wie insbesondere Methanol, Ethanol oder dergleichen, oder auch Ketone oder stärker polarisierbare Flüssigkeiten, wie Alkane.

Die auf diese Weise erhaltenen Produkte haben sich hinsichtlich ihrer pH-anhebenden Wirkung als am besten geeignet erwiesen. Zusätzlich ergab sich bei ihnen keine Tendenz des Zusammenbackens während des Mahlprozesses und beim Zusatz zu den kariogenen Nahrungs- und Genußmitteln.

Vorteilhafterweise verwendet man bei dem erfindungsgemäßen Verfahren als Ausgangsmaterial eine durch Fällung gewonnene und/oder vorvermahlene reine Erdalkaliverbindung.

Das folgende Beispiel dient der weiteren Erläuterung der Erfindung.

## Beispiel

Man beschickt eine Schwingmühle mit einem Fassungsvermögen von 15 l mit 6 kg eines vorgemahlenen Calcits mit einer spezifischen Oberfläche von 3 m²/g und spült mit Stickstoff. Dann schließt man die Mühle und setzt über eine Schleuse 35 g Ethanol zu. Nach dem Vermahlen während 120 Minuten wird das Mahlgut aus der Mühle entnommen und im Luftstrom bei einer Temperatur von etwa 60°C getrocknet. Man erhält ein hochdisperses Calcitpulver mit einer spezifischen Oberfläche von 12 m²/g und einer Gitterfehlerkonzentration, die sich in einer Halbwertsbreite der Röntgeninterferenzen von Cu-Kα-Strahlung im Winkelbereich von 20° bis 23° von 0,45 Winkelgraden widerspiegelt.

## Patentansprüche

1. Mittel zur Anhebung des pH-Werts der Zahnplaque, gekennzeichnet durch den Gehalt mindestens eines hochdispersen, schwerlöslichen Erdalkalioxids, Erdalkalihydroxids oder physiologisch unbedenklichen Salzes aus einem oder mehreren Erdalkalielementen und solchen Säuren, die mit Milchsäure ein Puffersystem bilden, dessen pH-Wert größer als 5,7 ist, jeweils mit einer spezifischen Oberfläche von mehr als 6 m²/g und einer Gitterfehlerkonzentration, die einer Halbwertsbreite der Röntgeninterferenzen von Cu-Kα Strahlung mit Glanzwinkelbereich von 20° bis 23° von mehr als 0,25 Winkelgraden entspricht.

2. Mittel nach Anspruch 1, dadurch gekennzeichnet, daß die Erdalkaliverbindung eine spezifische Oberfläche von mehr als 10 m²/g aufweist.

3. Mittel nach den Ansprüchen 1 oder 2, dadurch gekennzeichnet, daß die Erdalkaliverbindungen eine Gitterfehlerkonzentration aufweisen, die einer Halbwertsbreite der Röntgeninterferenzen von Cu-Kα-Strahlung im Glanzwinkelbereich von 20° bis 23° von mehr als 0,30 Winkelgraden entspricht.

4. Mittel nach einem der vorhergehenden Ansprüche, dadurch gekennzeichnet, daß es als Erdalkaliverbindung Oxide, Hydroxide oder Salze solcher Säuren, die mit Milchsäure ein Puffersystem bilden, dessen pH-Wert größer als 5,7 ist, wie insbesondere Carbonate, Silicate, Lactate, Tartrate und/oder Aluminate von Calcium und/oder Magnesium oder Doppelsalze aus diesen Verbindungen enthält.

5. Verfahren zur Herstellung der in dem Mittel nach den Ansprüchen 1 bis 4 enthaltenen hochdispersen Erdalkaliverbindungen, dadurch gekennzeichnet, daß die Erdalkaliverbindungen bis zum Erreichen einer Gitterfehlerkonzentration trocken vermahlen werden, die einer Halbwertsbreite der Röntgeninterferenzen von Cu-Kα-Strahlung im Glanzwinkelbereich von 20° bis 23° von mehr als 0,25 Winkelgraden entspricht.

6. Verfahren nach Anspruch 5, dadurch gekennzeichnet, daß das Vermahlen in einer Schwingmühle, einer Kugelmühle, einer Stiftmühle oder einer Prallmühle erfolgt.

7. Verfahren nach den Ansprüchen 5 oder 6, dadurch gekennzeichnet, daß das trockene Vermahlen in einer Atmosphäre bewirkt wird, die neben Luft oder Stickstoff die Dämpfe reiner polarer Flüssigkeiten mit einem Siedepunkt unterhalb 130°C oder von Mischungen solcher polarer Flüssigkeiten enthält.

8. Verfahren nach Anspruch 7, dadurch gekennzeichnet, daß die Mahlatmosphäre als verdampfte polare Flüssigkeit Wasser, einen niedermolekularen Alkohol und/oder ein Keton enthält.

9. Verfahren nach Anspruch 8, dadurch gekennzeichnet, daß als polare Flüssigkeit Ethanol eingesetzt wird.

10. Verfahren nach den Ansprüchen 5 bis 9, dadurch gekennzeichnet, daß als Ausgangsmaterial eine durch Fällung gewonnene oder vorvermahlene Erdalkaliverbindung eingesetzt wird.

## Claims

1. Agent for increasing the pH value of dental plaque characterized by the contact of at least one highly disperse, poorly soluble alkaline earth metal oxide, alkaline earth metal hydroxide or a physiologically acceptable salt of one or more alkaline earth metal elements and those acids which form, with lactic acid, a buffer system, the pH of which is greater than 5.7, in each case having a specific surface area greater than 6 $m^2/g$ and a concentration of lattice defects which corresponds to a half width of the X-ray peaks for $Cu-K_\alpha$ radiation at glancing angles from 20 to 23° of more tnan 0.25 degrees.

2. Agent according to Claim 1, characterized in that the alkaline earth metal compound has a specific surface area greater than 10 $m^2/g$.

3. Agent according to Claim 1 or 2, characterized in that the alkaline earth metal compound has a concentration of lattice defects which corresponds to a half width of the X-ray peaks for $Cu-K_\alpha$ radiation at glancing angles from 20 to 23° of more than 0.30 degrees.

4. Agent according to one of the preceding claims, characterized in that it contains, as the alkaline earth metal compound, oxides, hydroxides or salts of acids which, with lactic acid, form a buffer system whose pH value is greater than 5.7, in particular carbonates, silicates, lactates, tartrates and/or aluminates of calcium and/or magnesium, or double salts of these compounds.

5. Process for the preparation of a highly disperse alkaline earth metal compound present in the agent according to Claims 1 to 4, characterized in that the alkaline earth metal compound is dry-milled until the concentration of lattice defects which corresponds to a half width of the X-ray peaks of $Cu-K_\alpha$ radiation at glancing angles from 20° to 23° of more than 0.25 degrees is reached.

6. Process according to Claim 5, characterized in that milling is carried out in a vibratory mill, a ball mill, a pinned-disc mill or an impact mill.

7. Process accoring to Claim 5 or 6, characterized in that the dry-milling is effected in an atmosphere, which, in addition to air or nitrogen, contains the vapours of pure polar liquids having a boiling point below 130°C, or of mixtures of such polar liquids.

8. Process according to Claim 7, characterized in that the milling atmosphere contains water, a low molecular weight alcohol and/or a ketone as the vaporized polar liquid.

9. Process according to Claim 8, characterized in that ethanol is employed as the polar liquid.

10. Process according to Claims 5 to 9, characterized in that an alkaline earth metal compound obtained by precipitation or premilled is employed as the starting material.

**Revendications**

1. Agent d'élévation du pH de la plaque dentaire, caractérisé en ce qu'il contient au moins un constituant très dispersé peu soluble qui est un oxyde alcalino-terreux, un hydroxyde alcalino-terreux ou un sel physiologiquement acceptable d'un ou de plusieurs éléments alcalino-terreux et d'acides formant avec l'acide lactique un système tampon de pH supérieur à 5,7, chacun des constituants ayant une surface spécifique de plus de 6 $m^2/g$ et une concentration de défauts du réseau cristallin qui correspond à une largeur à mi-hauteur des interférences de rayons X du rayonnement $Cu_{K\alpha}$ égale à plus de 0,25 degré d'angle dans l'intervalle d'angles de brillance de 20° à 23°.

2. Agent selon la revendication 1, caractérisé en ce que le composé alcalino-terreux présente une surface spécifique de plus de 10 $m^2/g$.

3. Agent selon la revendication 1 ou 2, caractérisé en ce que les composés alcalino-terreux présentent une concentration de défauts du réseau cristallin qui correspond à une largeur à mi-hauteur des interférences de rayons X du rayonnement $Cu_{K\alpha}$ égale à plus de 0,30 degré d'angle dans l'intervalle d'angles de brillance de 20° à 23°.

4. Agent selon l'une des revendications précédentes, caractérisé en ce qu'il contient comme composés alcalino-terreux des oxydes, des hydroxydes ou des sels d'acides qui forment avec l'acide lactique un système tampon de pH supérieur à 5,7, comme, en particulier, des carbonates, des silicates, des lactates, des tartrates et/ou des aluminates de calcium et/ou de magnésium ou des sels doubles constitués de ces composés.

5. Procédé de fabrication des composés alcalino-terreux très dispersés contenus dans l'agent selon les revendications 1 à 4, caractérisé en ce que les composés alcalino-terreux sont broyés à sec jusqu'à ce que l'on atteigne une concentration de défauts du réseau cristallin qui correspond à une largeur à mi-hauteur des interférences de rayons X du rayonnement $Cu_{K\alpha}$ égale à plus de 0,25 degré d'angle dans l'intervalle d'angles de brillance de 20° à 23°.

6. Procédé selon la revendication 5, caractérisé en ce que le broyage a lieu dans un broyeur vibrant, un broyeur à boulets, un broyeur à barres ou un broyeur à impact.

7. Procédé selon les revendications 5 ou 6, caractérisé en ce que le broyage à sec est réalisé dans une atmosphère qui contient, outre l'air ou l'azote, les vapeurs de liquides polaires purs ayant un point d'ébullition inférieur à 130°C ou des mélanges de ces liquides polaires.

8. Procédé selon la revendication 7, caractérisé en ce que l'atmosphère de broyage contient comme liquides polaires vaporisés de l'eau, un alcool de faible poids moléculaire et/ou une cétone.

9. Procédé selon la revendication 8, caractérisé en ce que l'on utilise comme liquide polaire de l'éthanol.

10. Procédé selon les revendications 5 à 9, caractérisé en ce que l'on utilise comme matériau de départ un composé alcalino-terreux obtenu par précipitation ou prébroyé.